# EUROPÄISCHE PATENTANMELDUNG

(11) **EP 0 861 647 A2**
(43) Veröffentlichungstag der Anmeldung: **02.09.1998**
(21) Anmeldenummer: 98101347.7
(22) Anmeldetag: 27.01.1998
(51) Int. Cl.: A61F 13/66

(54) **Unterwäschesystem**

(30) Priorität: 30.01.1997 DE 19703336
(71) Anmelder: TAG Textilausrüstungs-Gesellschaft Schroers GmbH & Co. KG, 47805 Krefeld (DE)
(72) Erfinder: Goossens, Bernd, Dr., 47652 Weeze (DE); Geene, Hans-Bernd, 47608 Geldern (DE)
(74) Vertreter: Rehders, Jochen, Dipl.-Ing.

(57) **Zusammenfassung**

Unterwäschesystem zur Vermeidung der Folgen von Inkontinenz aus einer wiederverwendbaren Unterhose und einer auswechselbaren Absorbereinlage.

## Beschreibung

Die Erfindung betrifft ein Unterwäschesystem zur Vermeidung der Folgen von Inkontinenz aus einer wiederverwendbaren Unterhose mit einer auswechselbaren Absorbereinlage.

Das Problem der Inkontinenz, insbesondere bei älteren Menschen, ist bekannt. Um Urin und andere Körperausscheidungen von der Kleidung fernzuhalten und zurückzuhalten, werden absorbierende Artikel, wie Slipeinlagen oder "Windelhöschen" angeboten, die nach einmaligem Gebrauch entsorgt werden.

Aus der DE 44 07 808 A1 ist ein Unterwaschesystem bekannt, bei dem eine Unterhose aus einem Flachmaterial, welches einerseits eine Nässesperre bildet und andererseits Wasserdampf durchläßt, sowie wasch- und reinigungsbeständig ist, vorgesehen ist, wobei das Flachmaterial aus einem Verbundwerkstoff aus wenigstens einer Schicht aus einem nicht oder kaum in Wasser quellenden, hydrophilen, kompakten Polyurethan, wenigstens einer Schicht aus einem mikroporösen Polyurethan oder einem stark in Wasser quellenden, hydrophilen, kompakten Polyurethan, wenigstens einer Haftschicht aus einem stark in Wasser quellenden zweikomponentigen, hydrophilen, kompakten Polyurethan und einem Träger, der mit den Schichten flächig oder punktweise verbunden ist, besteht. Bei diesem bekannten Unterwäschesystem ist nachteilig, daß die hydrophile Schicht, die aus in Wasser quellbarem Polyurethan besteht, immer einen gewissen Anteil an Feuchtigkeit bindet und daher ein nasses Gefühl vermittelt, sowie aufgrund der Rücknässung unerwünschten Hautreaktionen Vorschub leistet.

Des weiteren ist aus der DE 41 18 609 A1 eine Sicherheitshose zur verwahrungssicheren Aufnahme von fluiden und gasförmigen Ausscheidungen von Darm und Blase bekannt, die zur Aufnahme größerer Ausscheidungsmengen geeignet sein, aus einem flüssigkeitsundurchlässigem, gummiartigen Material bestehen und gegenüber dem Körper vollständig abgedichtet sein soll und in der sich eine Einlage aus absorbierendem Material befindet. Diese Sicherheitshose ist für Extremfälle gedacht und ergibt aufgrund des völlig undurchlässigem Materials ein extrem unangenehmes Trageempfinden, und ist daher für die Benutzung durch Inkontinenzpatienten in weniger schweren Fällen nicht geeignet.

Die bekannten Hygieneartikel haben somit den Nachteil, daß sie entweder den Ansprüchen hinsichtlich Sicherheit gegen Durchtreten der Körperausscheidungen oder bezüglich Tragekomfort und bestmöglicher Wiederverwendbarkeit nicht genügen.

Der Erfindung liegt das Problem zugrunde, ein verbessertes Unterwäschesystem zur Vermeidung der Folgen von Inkontinenz zu schaffen, das größtmögliche Leckagesicherheit und Tragekomfort bietet und mit seinen wichtigsten Bestandteilen wiederverwendbar ist, so daß nur weniger aufwendige Bestandteile nach einmaligen Gebrauch zu entsorgen sind.

Ausgehend von dieser Problemstellung wird ein Unterwäschesystem vorgeschlagen, das erfindungsgemäß aus einer wiederverwendbaren Unterhose aus einem Dreischichten-Laminat, das eine Membranfolie als Zwischenschicht zwischen zwei textilen Flächengebilden enthält, wovon das körperseitige, textile Flächengebilde aus hydrophoben Faserkomponenten, und einer auswechselbaren Absorbereinlage, besteht.

Die Membranfolie ist dabei flüssigkeitsundurchlässig, atmungsaktiv und waschbeständig, insbesondere kochwaschbeständig, wobei als Materialien für eine kochwaschbeständige Membranfolie Folien auf Basis hydrophiler Polyether-Blockamide, mikroporöser Polyurethane oder nichtporöser, hydrophiler Polyurethane in Frage kommen. Zum Aufnehmen der zu bewältigenden Flüssigkeitsmenge ist in der Unterhose eine Absorbereinlage vorgesehen, die vorzugsweise im wesentlichen aus einer Fluff/Superabsorbermischung und einem Hüllvlies besteht. Die Fluff/Superabsorbermischung besteht üblicherweise aus fibrilierter Zellulose und vernetztem Polyacrylat in Partikelform. Aufgabe der Absorbereinlage ist es, die Flüssigkeit absorptiv zu fixieren, während durch die Unterhose überschüssige Flüssigkeitsmengen, die sich von der Absorbereinlage infolge einer zeitabhängigen Absorbercharakteristik nicht sofort binden lassen, sicher und ohne Durchnässen der darüber befindlichen Oberbekleidung zurückgehalten und der Absorbereinlage zugeleitet werden, wodurch dem Anwender aufgrund der Atmungsfähigkeit und der Elastizität ein optimaler Tragekomfort verschafft wird.

Bei den für die Unterhose verwendeten Dreischichten-Laminat ist zwischen zwei textilen Flächengebilden sandwichartig eine Membranfolie fixiert, die flüssigkeitsundurchlässig und dennoch atmungsaktiv und dabei waschbeständig, insbesondere kochwaschbeständig ist.

Die äußeren Schichten des Laminats können als Vlies, Gewirke oder Gewebe ausgebildet sein und weisen vorzugsweise eine Material- und/oder durch die Art des textilen Flächengebildes bedingte Bi-Elastizität auf, wodurch im Bauch und Beinbereich eine hohe Anschmiegsamkeit in Verbindung mit der gewünschten Abdichtungsfunktion gewährleistet ist. Die Bi-Elastizität ist bei Gewirken aufgrund der textilen Bindung gegeben, läßt sich jedoch ebenfalls durch einen Anteil an Elastofasern erreichen, die sich zu jeder Art von textilen Flächengebilden verarbeiten lassen und diesen textilen Flächengebilden eine erhöhte Elastizität, ggf. in Verbindung mit der Ausbildung des textilen Flächengebildes als Gewirke, erteilen.

Auf diese Weise wird mit dem erfindungsgemäßen Dreischichten-Laminat bereits im Bauch- und Beinbereich eine hohe Anschmiegsamkeit und damit eine gute Abdichtfunktion gewährleistet, jedoch können vorzugsweise die Ränder in diesen Bauch- und Beinbereichen noch zusätzlich mit einer elastischen Neoprenumsäumung versehen sein, wodurch der Sitz der Unterhose und die gewünschte Abdichtungsfunktion verbessert werden.

Das körperseitige, textile Flächengebilde aus hydrophoben Faserkomponenten, z. B. Polypropylen oder Polyester, leitet die Körperflüssigkeit optimal der Absorbereinlage zu und verhindert eine Rücknässung.

Die Unterhose des erfindungsgemäßen Unterwäschesystems ist beliebig wiederverwendbar, da das Dreischichten-Laminat kochwaschbeständig ist, ohne seine vorteilhaften Eigenschaften zu verlieren.

Die Absorbereinlage ist eine Slipeinlage, die als wesentlichen Bestandteil eine Fluff/Superabsorbermischung enthält und von einem Hüllvlies umgeben ist. Diese Absorbereinlage läßt sich vorteilhafterweise mittels eines Klebestreifens an der Innenseite der Unterhose auswechselbar befestigen.

Die Erfindung wird nachstehend anhand eines in der Zeichnung dargestellten Ausführungsbeispiels des näheren erläutert. In der Zeichnung zeigen:

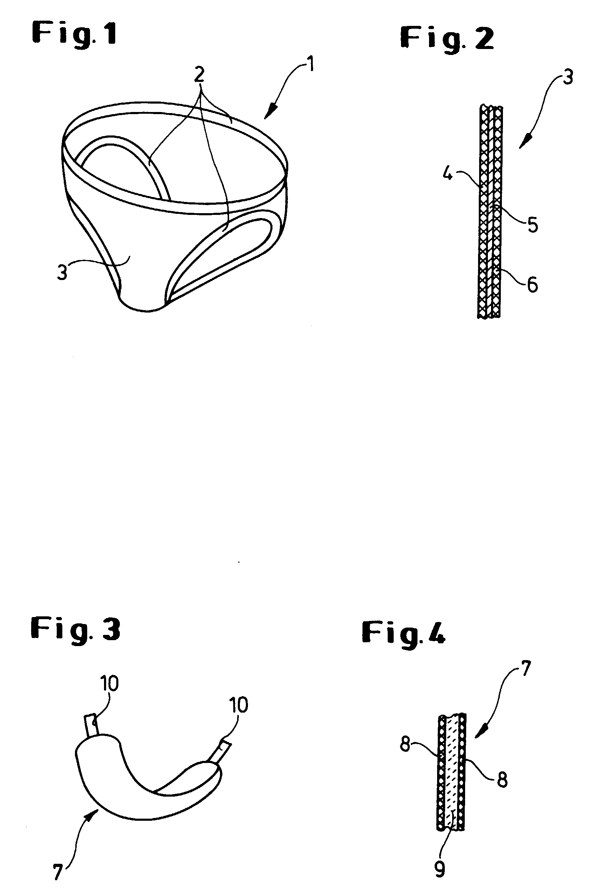

- Fig. 1: eine perspektivische Ansicht einer Unterhose des erfindungsgemäßen Unterwäschesystems,
- Fig. 2: einen Schnitt durch das textile Flächengebilde der Unterhose,
- Fig. 3: eine perspektivische Ansicht einer in der Unterhose gemäß Fig. 1 zu tragenden Absorbereinlage und
- Fig. 4: einen Querschnitt durch die Absorbereinlage gemäß Fig. 3.

Eine Unterhose 1 ist aus einem Dreischichten-Laminat 3 hergestellt und im Bauch- und Beinbereich mit einer elastischen Neoprenumsäumung 2 versehen.

Das Dreischichten-Laminat besteht aus einem körperseitigen, textilen Flächengebilde aus hydrophoben Faserkomponenten wie z. B. Polypropylen oder Polyester, um Körperflüssigkeit optimal einer Absorbereinlage 7 zuzuleiten und eine Rücknässung zu verhindern. Zwischen diesem körperseitigen, textilen Flächengebilde 4 und einem äußeren, textilen Flächengebilde 6 ist eine flüssigkeitsundurchlässige, atmungsaktive Membran 5 angeordnet. Bei dieser Membran handelt es sich beispielsweise um eine mikroporöse Polyurethanmembran, die für Flüssigkeiten undurchlässig ist, Gase und Dämpfe jedoch durchläßt, wodurch das Dreischichten-Laminat eine hohe Leckagesicherheit bei bestem Tragekomfort und sehr häufiger Wiederverwendbarkeit ergibt. Ebenso sind Membranfolien auf Basis hydrophiler Polyether-Blockamide oder hydrophiler, nichtporöser Polyurethane verwendbar. Die textilen Flächengebilde 4, 6 können als Vlies, Gewirke oder Gewebe hergestellt sein und weisen vorzugsweise eine hohe Bi-Elastizität auf, um im Bauch- und Beinbereich eine gute Anschmiegsamkeit zu erreichen. Die Bi-Elastizität kann durch die Art des textilen Flächengebildes, insbesondere, wenn Gewirke verwendet werden, erreicht werden. Auch ein Anteil an Elastofasern in den textilen Flächengebilden 4, 6 bewirkt die erwünsche Bi-Elastizität, ggf. in Verbindung mit einer entsprechenden Art des textilen Flächenbildes.

Durch das Dreischichten-Laminat 3 der Unterhose 1 werden überschüssige Flüssigkeitsmengen, die von der Absorbereinlage 7 infolge einer zeitabhängigen Absorbercharakteristik nicht sofort verbunden werden können, sicher und ohne Durchnässen der darüber befindlichen Oberbekleidung zurückgehalten, der Absorbereinlage zugeleitet und Verschaffen dem Träger aufgrund der Atmungsfähigkeit und Elastizität einen optimalen Tragekomfort.

Die Absorbereinlage 7 ist wie eine Slipeinlage aus einer Fluff/Superabsorbermischung 9 als wesentlichen Bestandteil, das von einem Hüllvlies 8 umgeben ist, ausgebildet. Zum Fixieren der Absorbereinlage 7 in der Unterhose 1 sind daran Klebestreifen 10 angebracht.

## Patentansprüche

1. Unterwäschesystem zur Vermeidung der Folgen von Inkontinenz, bestehend aus einer wiederverwendbaren Unterhose (1) aus einem Dreischichten-Laminat (3), das eine Membranfolie (5) als Zwischenschicht zwischen zwei textilen Flächengebilden (4, 6) enthält, wovon das körperseitige, textile Flächengebilde aus hydrophoben Faserkomponenten besteht, und einer auswechselbaren Absorbereinlage (7).

2. Unterwäschesystem nach Anspruch 1, **dadurch gekennzeichnet**, daß die Membranfolie (5) flüssigkeitsundurchlässig, atmungsaktiv und waschbeständig, insbesondere kochwaschbeständig ist.

3. Unterwäschesystem nach Anspruch 1 oder 2, **dadurch gekennzeichnet**, daß das Dreischichten-Laminat (3) bi-elastisch ist.

4. Unterwäschesystem nach einem oder mehreren der Ansprüche 1 bis 3, **dadurch gekennzeichnet**, daß zur elastischen Abdichtung der Unterhose (1) an den Rändern (Bein- und Bauchbereich) elastische Neoprenumsäumungen (2) verwendet werden.

5. Unterwäschesystem nach einem oder mehreren der Ansprüche 1 bis 4, **dadurch gekennzeichnet**, daß die Absorbereinlage (7) im wesentlichen aus einer Fluff/Superabsorbermischung (9) und einem Hüllvlies (8) besteht.

6. Unterwäschesystem nach Anspruch 5, **dadurch gekennzeichnet**, daß die Absorbereinlage (7) zur Fixierung an der Innenseite der Unterhose (1) mit Klebestreifen (10) ausgerüstet ist.

7. Unterwäschesystem nach einen oder mehreren der Ansprüche 1 bis 6, **dadurch gekennzeichnet**, daß als Membranfolie (5) kochwaschbeständige Folien auf Basis hydrophiler Polyether-Blockamide, mikroporöser Polyurethane oder hydrophiler, nichtporöser Polyurethane verwendet werden.
